Europäisches Patentamt

European Patent Office

Office européen des brevets

(19)

(11) Publication number: **0 126 650**
**A2**

(12)

# EUROPEAN PATENT APPLICATION

(21) Application number: **84303439.8**

(51) Int. Cl.³: **A 61 M 1/03**

(22) Date of filing: **21.05.84**

(30) Priority: **23.05.83 GB 8314189**

(71) Applicant: **Japan Medical Supply Company Limited, 12-17 Kako-Machi Naka-ku, Hiroshima City Hiroshima Pref. (JP)**

(43) Date of publication of application: **28.11.84 Bulletin 84/48**

(72) Inventor: **Clark, Russell Donald, Dr., United Christian Hospital 130 Hip Wo Street, Kwun Tong Kowloon (HK)**
Inventor: **Hua Su Ping, Andrew, 27 Sackville Street, Kew Victoria 3101 (AU)**

(84) Designated Contracting States: **BE CH DE FR GB IT LI NL SE**

(74) Representative: **Allen, Oliver John Richard et al, Lloyd Wise, Tregear & Co. Norman House 105-109 Strand, London, WC2R 0AE (GB)**

(54) **Technique and equipment for dialysis treatment.**

(57) A technique for connecting a catheter to a dialysis device is provided with the dialysis device comprising a dialysate solution bag, a connecting tube, one end of which is sealingly joined to the dialysate solution bag and the other end of which is sealingly connected into a flexible, substantially transparent sterilising bag containing a sterilising medium, the sterilising bag being additionally provided with a sealed opening through which the external end of an implanted catheter or the free end of an intermediate tube (the other end of which is connected to the external end of an implanted catheter) can penetrate into the sterilising bag to complete the connection from the dialysate solution bag to the patient.

The technique and device help reduce the incidence of peritonitis in patients undergoing continuous ambulatory peritoneal dialysis.

ACTORUM AG

# TECHNIQUE AND EQUIPMENT FOR DIALYSIS TREATMENT

The present invention relates to a technique and equipment to ensure a sterile environment is maintained during all stages of the connection of medical equipment to the human body such as peritoneal dialysis equipment for the treatment of renal diseases or kidney failure.

A technique known as continuous ambulatory peritoneal dialysis (CAPD) is an accepted but not widespread form of treatment for patients with end-stage renal disease or kidney failure. This technique was developed by Popovich and Moncrief in Texas in 1976 and is now used worldwide with some 13,000 people using this form of treatment at present.

However, there is a serious disadvantage of such treatment namely peritonitis which is an inflammation inside the peritoneal cavity or abdomen caused by the presence of bacteria in what is normally a sterile part of the body. In CAPD, bacteria may become present in this normally sterile part of the body because the normally enclosed peritoneal cavity is open to the environment as a catheter is permanently implanted into the cavity to allow for dialysis or fluid exchange to take place. Bacterial access to the peritoneal cavity is facilitated each time the catheter is connected to the peritoneal dialysis bags.

For each patient, this happens four times day, six times a week or 1,248 times a year.

It has been estimated that peritonitis can occur with a frequency of from one episode per ten patient weeks to one episode per two patient years. Peritonitis is painful, inconvenient and expensive to treat and in the ultimate can cause so much damage that peritoneal dialysis cannot be used and may even be fatal.

Any method or apparatus capable of reducing to a minimum the incidence of peritonitis would increase the usage of CAPD as an acceptable method of treating kidney failure. Since CAPD is potentially much cheaper than the more orthodox form of treatment using haemodialysis, this is desirable particularly in third world countries where the cost of haemodialysis equipment is prohibitive.

To prevent peritonitis as a result of CAPD, it is essential to prevent bacterial entry into the peritoneal cavity at the time of connection.

In one of the existing equipments on the market for CAPD, i.e. that supplied by Travenol, the supply of CAPD fluid is in a sealed plastic bag with two tubing outlets sealed by a diaphragm or a membrane, connection to the catheter being made via a length of plastic tubing. To connect the CAPD fluid bag to the catheter implanted in the patient, two separate connecting steps have to be made, one from one end of the connecting tube to the CAPD bag and one from the other end of the connecting tubing to the implanted catheter, i.e. there are two opportunities for bacteria to enter the peritoneal cavity. In practice, modifications and adaptations have been made to reduce the risk of infection entering at the points of connection such as the use of a titanium adaptor semi-permanently connecting the catheter to the tubing. By semi-permanently is meant that the titanium adaptor can be kept connected to the catheter and tubing for up to one month.

The other end of the tubing is provided with a stylet which can then either be simply pushed through the diaphragm of the CAPD bag into the CAPD bag after chemically sterilising the stylet end of the tubing. Practice and skill makes this type of connection quick and efficient but there is still a risk of bacterial penetration as in essence each connection is being made in an open environment where bacteria exist and so there is a risk that bacteria may be introduced into the CAPD bag containing the dialysate which is an ideal medium for bacterial growth. Also, a pulse of air is introduced at each connection and air around people contains bacteria.

In a modification of this method, a plastic container which contains a sponge impregnated with a sterilant - povidone-iodine - is placed around the stylet as it is joined to the tubing of the CAPD bag. The plastic container snaps shut thus providing an antiseptic environment in which the connection can take place.

0126650

- 4 -

Although this modification is an improvement, bacterial contamination of the stylet still occurs in the air and ordinary air still surrounds the connection and again a pulse of air is introduced into the system.

In other techniques, filters have been incorporated into the connecting tubes (Charles Mion France) but this has not been done on a commercial scale. To filter out bacteria, the pore size of the filter must be very small and consequently pressure is needed to push the fluid through the filter and so renders this technique unsuitable as it considerably lengthens the period of fluid exchange which should ideally be less than one hour.

We have sought to provide an apparatus and technique to ensure that connection of the implanted catheter to the CAPD bag takes in a sterile atmosphere.

Accordingly, in one aspect the present invention provides a peritoneal dialysis device comprising a dialysate solution bag, a connecting tube, one end of which is sealingly joined to the dialysate solution bag and the other end of which is sealingly connected into a flexible, substantially transparent sterilising bag containing a sterilising medium, the sterilising bag being additionally provided with a sealed opening through which the external end of an implanted catheter or the free end of an intermediate tube (the other end of which is connected to the external end of an implanted catheter)

can penetrate into the sterilising bag to complete the connection from the dialysate solution bag to the patient.

In another aspect, the present invention provides a peritoneal dialysis device comprising a dialysate solution bag, a connecting tube, one end of which is sealingly joined to the dialysate solution bag and the other end of which is sealingly connected into a flexible, substantially transparent, sterilising bag containing a sterilising medium, the sterilising bag being additionally provided with a sealed opening through which the external end of an implanted catheter can penetrate into the sterilising bag to complete the connection from the dialysate solution bag to the patient.

However, rather than connecting the external end of the catheter directly into the sterilising bag, an intermediate tube may be connected to the free end of the implanted catheter and the free end of the intermediate tube is inserted into the sterilising bag through the opening.

Such a sealed opening can be provided by any suitable means so long as it remains sealed at all times, including during autoclaving, until actually used. A simple scored line which separates on bending is an easy method. Alternatively the sterilising bag may be provided with two flaps or tabs which are fused together but can be readily separated by manually pulling them apart.

The sterilising medium is preferably gauze soaked in an antiseptic such as 1% chlorhexidine (hibitane) in water. 1% hibitane in water is preferred because it can be autoclaved and is a quick acting broad spectrum antiseptic.

The connecting tube is preferably connected to the sterilising bag by passing it through an inlet slit in the sterilising bag and welding it to seal it into position, the inlet slit having previously been used to insert the sterilising medium into the sterilising bag.

After the dialysate solution bag, tubing and sterilising bag have been joined together the whole is sterilised in an autoclave.

Preferably, the sterilising bag is ovoid in shape with a long axis of approximately 16cm and is made from medical grade polyvinyl chloride 0.13mm thick. The outlet slit to receive the catheter is preferably about 19mm long and the inlet, before welding 2.5cm long.

The advantages of the above device is that the connecting tube is sealed to the bag containing the dialysate solution and so when the patient is connected for dialysis only one connection step is required, i.e. directly or indirectly to the implanted abdominal catheter near the patient's body and this connection takes place within an enclosed sterile environment so that there is no possibility of bacterial contamination by either accidental contact or via a pulse of air delivered at the time of connection.

The device of the present invention can be used with presently available catheters either inserting the external end of same directly into the sterilising bag or connecting the external end of same to the intermediate tube which is then inserted into the sterilising bag.

In another aspect, the present invention provides a method of connecting a catheter to a dialysis device, of the type described above, to ensure sterility at all stages comprising the steps of:

(a) opening the outlet slit of the sterilising bag of the dialysis device and inserting the capped end of the catheter or intermediate tube into the sterilising bag,

(b) closing the outlet slit to sealingly secure the capped end of the catheter or intermediate tube in the sterilising bag,

(c) maintaining the capped end of the catheter or intermediate tube in contact with the sterilising medium in the sterilising bag for the required period,

(d) removing the cap from the end of the catheter or intermediate tube and, if one is provided, from the end of the connecting tube and maintaining the ends of the catheter or intermediate tube and the connecting tube in contact with the sterilising medium for the required sterilising period, and

(e)     joining the connecting tube to the catheter or intermediate tube.

In yet a further aspect of the present invention, there is provided a method of connecting a catheter to a dialysis device of the type described above to ensure sterility at all stages comprising the steps of:

(a)     opening the outlet slit of the sterilising bag of the dialysis device and inserting the capped end of the catheter into the sterilising bag,

(b)     closing the outlet slit to sealingly secure the capped end of the catheter in the sterilising bag,

(c)     maintaining the capped end of the catheter in contact with the sterilising medium in the sterilising bag for the required period,

(d)     removing the cap from the end of the catheter and, if one is provided, from the end of the connecting tube and maintaining the ends of the catheter and the connecting tube in contact with the sterilising medium for the required sterilising period, and

(e)     joining the connecting tube to the catheter.

In the above-described method, it is preferred to use an effective, quick acting antiseptic to induce sterility within the sterilising bag and of the contaminated ends of the catheter and connecting tubes enclosed within the plastic bag. Each sterilising period generally takes five minutes. By using a transparent flexible plastic to form the sterilising bag, manipulation of the various parts to be sterilised is visually facilitated. When the sterilising medium comprises a hibitane solution impregnated gauze, each sterilising step involves wrapping the gauze around the part being sterilised, e.g. the capped catheter, the uncapped catheter and connecting tube for five minutes. After connection of the catheter and the connecting tube, the capped catheter can be further sterilised ready for recapping the catheter after dialysis by being immersed in the sterilising medium, e.g. well wrapped in the hibitane solution impregnated gauze.

Generally for convenience during use, the sterilising bag is taped around the catheter.

The invention is further illustrated in the accompanying drawing in which:

Figure 1 is a cross-section through part of a peritoneal dialysis device in accordance with the present invention; and

Figure 2 shows the steps involved in connecting a catheter to a dialysis device in accordance with the present invention.

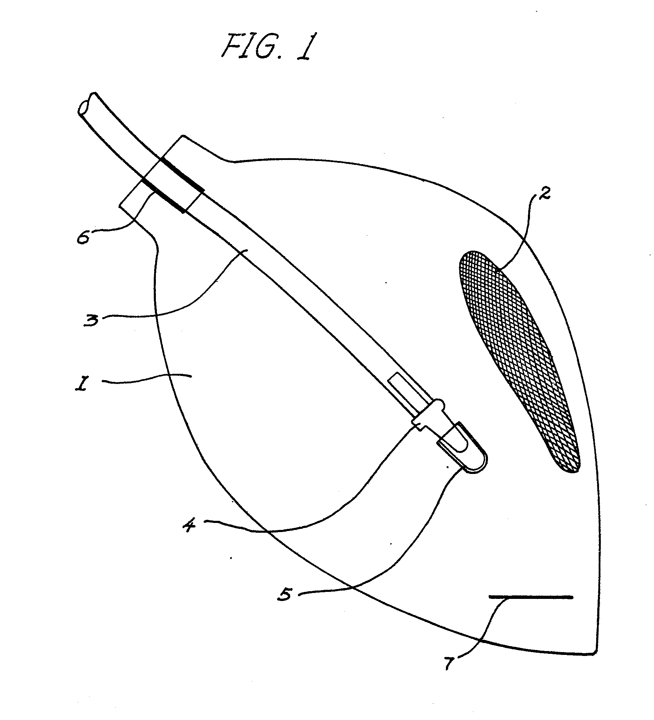

Figure 1 shows part of a peritoneal dialysis device of the present invention which comprises a transparent sterilising bag 1 made of medical grade polyvinyl chloride 0.13mm in thickness. Inside the sterilising bag 1 is a sterilising medium 2 comprising one or more pieces of gauze (2-inch square) soaked in a solution of 1% chlorhexidine (hibitane) in water. Sealingly contained within the sterilising bag 1 is a connecting tube 3, the end 4 of which is provided with a cap 5. The other end of the connecting tube 3 is connected to a bag containing the dialysate solution (not shown). The connecting tube 3 is sealed into the sterilising bag 1 by means of a weld 6. The sterilising bag 1 is also provided with a sealed opening 7 through which a catheter or intermediate tube (not shown) can pass to be connected to the end 4 of the connecting tube 3.

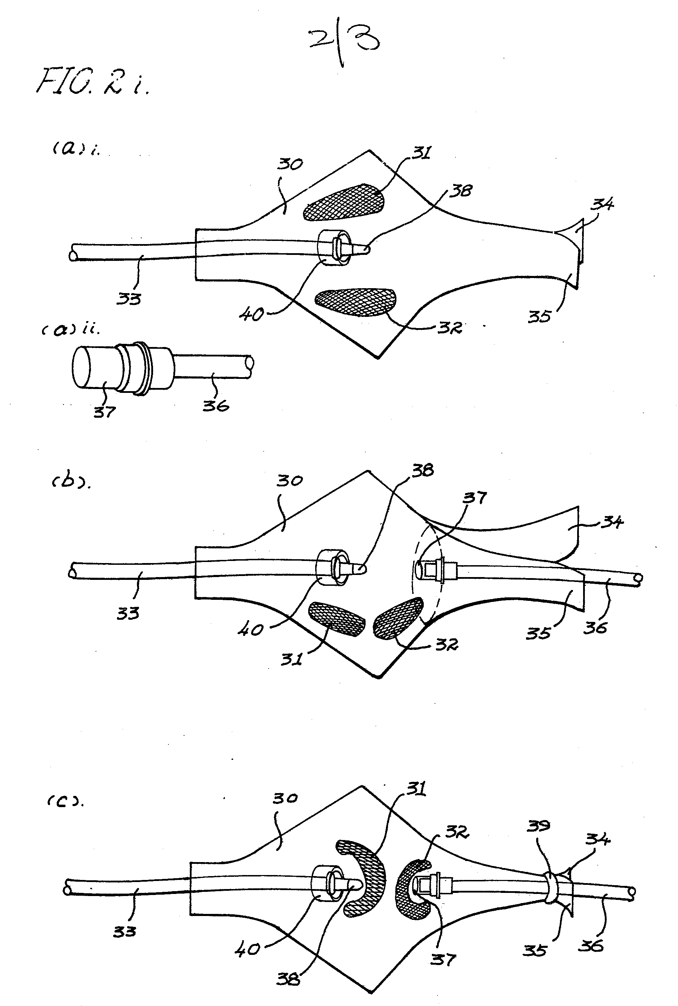

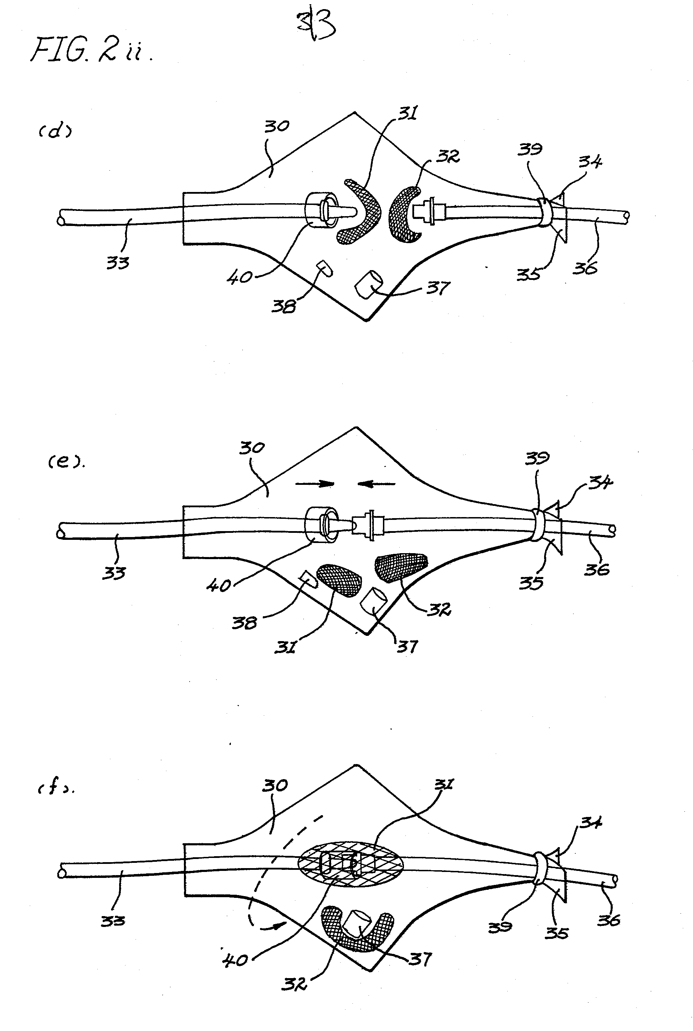

Figure 2 shows a transparent sterilising bag 30 made of the same type of material as the bag 1 of Figure 1. Inside the bag 30 are two pieces of gauze 31 and 32 soaked in a sterilising medium of a solution of 1% chlorhexidine in water. Sealing contained within the sterilising bag 30 is one end of a connecting tube 33 covered with a cap 38 and is provided with a locking device 40. The other end of the connecting tube 33 is connected to a bag containing the dialysate solution. The sterilising bag 30 is also provided with tabs 34 and 35 which are sealed to one another but can be separated by being pulled apart to permit the entry into the sterilising bag 30 of the end of an extension tube 36, the other end of which is connected to a catheter (not shown) implanted in a patient's body. The end of the extension tube 36 is covered with a screw cap 37.

To connect the catheter to the bag containing the dialysate via the sterilising bag 30 involves the following steps:

1) Tabs 34 and 35 are pulled apart and the extension tube 36 is inserted in the sterilising bag 30.

2) A clamp (not shown) is applied to the extension tube 36 and the tabs 34 and 35 are wrapped around the extension tube 36 and held in place by tape 39.

3) The antiseptic gauzes 31 and 32 are wrapped around the ends of the connecting tube 33 and extension tube 36 respectively and left in position for five minutes.

4) The screw cap 37 is removed from the end of the extension tube 36 and the cap 38 from the end of the connecting tube 33 and the gauzes 31 and 32 are repositioned over the exposed ends of the connecting tube 33 and the extension tube 36, respectively, and left in position for a further five minutes.

5) The pieces of gauze 31 and 31 are removed from the exposed ends of the connecting tube 33 and the extension tube 36 and these exposed ends of the connecting tube 33 and the extension tube 36 are pushed together and held in that position by means of the locking device 40.

6) The joined ends of the connecting tube 33 and the extension tube 36 are wrapped in the piece of gauze 31 and the screw cap 37 is wrapped in the piece of gauze 32.

The connection is now complete and dialysis can proceed by removing the clamp from the extension tube 36.

WHAT WE CLAIM IS:

1.    A peritoneal dialysis device comprising a dialysate solution bag, a connecting tube, one end of which is sealingly joined to the dialysate solution bag and the other end of which is sealingly connected into a flexible, substantially transparent sterilising bag containing a sterilising medium, the sterilising bag being additionally provided with a sealed opening through which the external end of an implanted catheter or the free end of an intermediate tube (the other end of which is connected to the external end of an implanted catheter) can penetrate into the sterilising bag to complete the connection from the dialysate solution bag to the patient.

2.    A peritoneal dialysis device comprising a dialysate solution bag, a connecting tube, one end of which is sealingly joined to the dialysate solution bag and the other end of which is sealingly connected into a flexible, substantially transparent, sterilising bag containing a sterilising medium, the sterilising bag being additionally provided with a sealed opening through which the external end of an implanted catheter can penetrate into the sterilising bag to complete the connection from the dialysate solution bag to the patient.

3.    A peritoneal dialysis device as claimed in Claim 1 or 2 in which the sealed opening is a scored line.

4. A peritoneal dialysis device as claimed in any of claims 1 to 3 in which the sterilising medium is 1% chlorhexidine in water.

5. A peritoneal dialysis device as claimed in any of claims 1 to 4 in which the connecting tube is inserted into the sterilising bag through a slit therein and thereafter welded to the sterilising bag.

6. A peritoneal dialysis device as claimed in any of claims 1 to 5 in which the sterilising bag is ovoid with a long axis of approximately 16 cm.

7. A peritoneal dialysis device as claimed in any of claims 1 to 6 in which the sterilising bag is made of medical grade polyvinyl chloride of 0.13 mm thickness.

8. A method of connecting a catheter to a dialysis device, of the type claimed in any of the preceding claims, to ensure sterility at all stages comprising the steps of:

    (a) opening the outlet slit of the sterilising bag of the dialysis device and inserting the capped end of the catheter or intermediate tube into the sterilising bag,

    (b) closing the outlet slit to sealingly secure the capped end of the catheter or intermediate tube in the sterilising bag,

    (c) maintaining the capped end of the catheter or intermediate tube in contact with the sterilising medium in the sterilising bag for the required period,

(d) removing the cap from the end of the catheter or intermediate tube and, if one is provided, from the end of the connecting tube and maintaining the ends of the catheter or intermediate tube and the connecting tube in contact with the sterilising medium for the required sterilising period, and

(e) joining the connecting tube to the catheter or intermediate tube.

9.     A method of connecting a catheter to a dialysis device, of the type claimed in Claim 2, to ensure sterility at all stages comprising the steps of:

(a) opening the slit of the sterilising bag of the dialysis device and inserting the capped end of the catheter into the sterilising bag,

(b) closing the outlet slit to sealingly secure the capped end of the catheter in the sterilising bag,

(c) maintaining the capped end of the catheter in contact with the sterilising medium in the sterilising bag for the required period,

(d) removing the cap from the end of the catheter and, if one is provided, from the end of the connecting tube and maintaining the ends of the catheter and the connecting tube in contact with the sterilising medium for the required sterilising period, and

(e) joining the connecting tube to the catheter.

1/3

FIG. 1

FIG. 2 i.

(a) i.

(a) ii.

(b).

(c).

33

FIG. 2 ii.

(d)

(e).

(f).